# EUROPÄISCHE PATENTSCHRIFT

(11) **EP 2 712 633 B1**
(45) Veröffentlichungstag und Bekanntmachung des Hinweises auf die Patenterteilung: **29.04.2015**
(21) Anmeldenummer: 13178872.1
(22) Anmeldetag: 01.08.2013
(51) Int. Cl.: A61L 27/36, A61L 33/00, A61L 33/06

(54) **Bioprosthetische Komponenten für ein Implantat, insbesondere teilvernetzte biologische Herzklappen**
Bioprosthetic components for an implant, in particular partly crosslinked biological heart valves
Composants bioprothétiques pour un implant, notamment valves cardiaques biologiques partiellement réticulées

(30) Priorität: 02.10.2012 US 201261708647 P
(43) Veröffentlichungstag der Anmeldung: 02.04.2014
(73) Patentinhaber: Biotronik AG, 8180 Bülach (CH)
(72) Erfinder: Borck, Alexander, 91086 Aurachtal (DE)
(74) Vertreter: Lindner-Vogt, Karin L.

(56) Entgegenhaltungen:
- WO-A1-01/28604
- WO-A1-01/97874
- WO-A1-2006/066327
- DE-A1- 2 715 466
- US-A1- 2002 086 977
- US-A1- 2003 028 247
- PAÉZ J M GARCÍA ET AL: "Resistance and stability of a new method for bonding biological materials using sutures and biological adhesives", JOURNAL OF BIOMATERIALS APPLICATIONS, TECHNOMIC, LANCASTER, PA, US, Bd. 19, Nr. 3, Januar 2005 (2005-01), Seiten 215-236, XP008166429, ISSN: 0885-3282, DOI: 10.1177/0885328205046633
- DATABASE MEDLINE [Online] US NATIONAL LIBRARY OF MEDICINE (NLM), BETHESDA, MD, US; 1981, JONES J G ET AL: "The quantification of zinc in the mucosal cells of human small intestine using x-ray microanalysis.", XP002718233, Database accession no. NLM6947400 & JONES J G ET AL: "The quantification of zinc in the mucosal cells of human small intestine using x-ray microanalysis.", SCANDINAVIAN JOURNAL OF GASTROENTEROLOGY. SUPPLEMENT 1981, Bd. 70, 1981, Seiten 37-48, ISSN: 0085-5928
- José Páez ET AL: "Mechanical resistance of a new biomaterial, ostrich pericardium, and a new method of joining tissues combining suturing and a biological adhesive", Journal of Biomedical Materials Research Part B: Applied Biomaterials, 15. Oktober 2004 (2004-10-15), Seiten 9-16, XP055094617, Hoboken DOI: 10.1002/jbm.b.30139 Gefunden im Internet: URL:http://www.ncbi.nlm.nih.gov/pubmed/154 90469 [gefunden am 2013-12-20]
- DATABASE MEDLINE [Online] US NATIONAL LIBRARY OF MEDICINE (NLM), BETHESDA, MD, US; Januar 2000 (2000-01), VASUDEV S C ET AL: "The anticalcification effect of polyethylene glycol-immobilized on hexamethylene diisocyanate treated pericardium.", XP002721519, Database accession no. NLM10676579 & VASUDEV S C ET AL: "The anticalcification effect of polyethylene glycol-immobilized on hexamethylene diisocyanate treated pericardium.", ARTIFICIAL CELLS, BLOOD SUBSTITUTES, AND IMMOBILIZATION BIOTECHNOLOGY JAN 2000, Bd. 28, Nr. 1, Januar 2000 (2000-01), Seiten 79-94, ISSN: 1073-1199

## Beschreibung

Die vorliegende Erfindung betrifft bioprosthetische Komponenten basierend auf oder umfassend biologische Materialien für Implantate, vorzugsweise biologische Herzklappen, insbesondere biologische Herzklappensegel, die nur an mechanisch beanspruchten Stellen chemisch stabilisiert (teilvernetzt) wurden und somit Zonen mit unterschiedlichen mechanischen Eigenschaften aufweisen, sowie ein Verfahren zu deren Herstellung.

Für Implantate werden im Stand der Technik auch zunehmend bioprosthetische Komponenten in Betracht gezogen, die auf einem nativen oder ggf. modifizierten biologischen Material basieren oder biologischen Materialien als Komponente umfassen. Üblicherweise wird die Oberfläche des biologischen Materials, das als bioprosthetische Komponente für ein Implantat dienen soll, vor der Verwendung einer stabilisierenden Behandlung unterworfen. Häufige Beispiele für die Anwendung solcher Implantate sind z.B. diverse Herzklappen wie z.B. Herzsegel, Aortenklappe, Mitralklappe, Pulmonarklappe. Weitere Beispiele für die Anwendung solcher Implantate sind z.B. Venenklappen und sog. Closure Devices wie Aneuryismen-Stents, die im gesamten Blutkreislauf einschließlich des Himbereichs zum Einsatz kommen können, sowie Herzsegel zum Abdichten von Narben, die z.B. durch eine Intervention am Herzen, beispielsweise die Behebung einer genetisch bedingten Öffnung zwischen Herzkompartimenten, bedingt sein können. Die durch einen Gendefekt hervorgerufene Öffnung zwischen Herzkompartimenten, welche häufig schon im Kindesalter behandlungspflichtig ist, kann z.B. ein atrioventrikulärer Septumdefekt, ein Atriumseptumdefekt oder ein Ventrikelseptumdefekt sein.

Bei Säugetieren ist das Herz das Organ, das für die Aufrechterhaltung einer angemessenen Versorgung verantwortlich ist, indem es Blut durch den gesamten Körper pumpt, damit alle Teile des Körpers ausreichend mit Sauerstoff und Nährstoffen versorgt werden. Das Zurückströmen von Blut in das Herz wird durch vier Klappen (Herzklappen), die als Ein- und Ausgang für jede der beiden Herzkammern, die als Pumpkammern des Herzens dienen, verhindert.

Eine Fehlfunktion eines oder mehrerer dieser Ventile kann schwerwiegende gesundheitliche Folgen nach sich ziehen. Solche Fehlfunktionen können durch angeborene Missbildungen oder Krankheiten-bedingte Schäden verursacht werden. Zu den Formen von Fehlfunktionen gehören Stenose (Verengung in der Mündung des geöffneten Ventils) und das Zurückfließen des Bluts durch die Schließung oder das geschlossene Ventil, wobei beide eine erhöhte Leistung des Herzens erfordern, um den entsprechenden Blutfluss in den Körper aufrechtzuerhalten. In vielen Fällen besteht die einzig wirksame Lösung darin, das eine Fehlfunktion aufweisende Ventil zu ersetzen.

Die Verwendung künstlicher Herzklappen erfordert leider, dass die Patienten lebenslang mit Antikoagulantien behandelt werden müssen, da sich sonst Blutgerinnsel auf dem Ventil der künstlichen Herzklappe bilden können. Blutgerinnsel auf dem Ventil können entweder die Beweglichkeit der Ventilöffnungsteile einschränken, die Ventilfunktion beeinträchtigen oder sich vom Ventil ablösen und die Blutgefäße hinter dem Ventil verschließen. Bei mechanischen Ventilen dreht sich das Verschlusselement in der Durchflussöffnung, wird aber nicht von der Durchflussöffnung wegbewegt, wenn das Ventil öffnet. Dies stellt eine Einschränkung für die Strömung des Blutes dar, aber was noch wichtiger ist, es stört die Durchblutungsmuster (blood flow patterns). Diese Störung des Blutflusses wird allgemein als Ursache angesehen, oder liefert zumindest einen wesentlichen Beitrag, zur beobachteten Neigung der mechanischen Ventile eine Blutgerinnung zu verursachen.

Biologische Prothesen, z.B. biologische Ersatzherzklappen, die aus natürlichen Geweben gewonnen werden, können aufgrund bestimmter klinischer Vorteile gegenüber mechanischen Vorrichtungen bevorzugt sein. Beispielsweise ist bei Gewebe-basierten Prothesen in der Regel die Routine-Antikoagulation nicht erforderlich und während mechanische Prothesen typischerweise plötzlich versagen können, tritt auf der anderen Seite bei Gewebe-basierten Prothesen in der Regel erst eine allmähliche Verschlechterung auf, die sich über einen Zeitraum von Monaten oder sogar Jahren erstrecken kann und somit frühzeitige Hinweise auf ein mögliches Versagen liefert. Neben künstlichen Herzklappen werden daher bei bestimmten Patientengruppen, z.B. bei denen die Implantation künstlicher Herzklappen ausscheidet, auch biologische Herzklappen als Ersatz für Herzklappen mit einer Fehlfunktion verwendet.

Zwar ist die Wahrscheinlichkeit einer Blutgerinnung bei biologischen Herzklappen gegenüber mechanischen Ersatzventilen deutlich geringer, und Patienten mit biologischen Herzklappen müssen somit in der Regel, mit Ausnahme der unmittelbaren postoperativen Periode, nicht mit Antikoagulantien behandelt werden, dennoch weisen auch die bekannten biologischen Herzklappen noch Verbesserungsbedarf auf. So können biologische Herzklappen im Laufe der Zeit nachlassen, oft als Folge einer Mineralisierung bzw. einer Verkalkung des vernetzten natürlichen Gewebes, was vor allem bei jungen Patienten ein gravierendes Problem darstellt. Obwohl also Empfänger einer biologischen Herzklappe kein Antikoagulans wie z.B. Marcumar nehmen müssen, ist die Haltbarkeit von biologischen Herzklappen geringer als die von mechanischen Ventilen.

Obwohl jede Prothesenklappe aufgrund von Mineralisierung wie z.B. Verkalkung versagen kann, ist dieses Problem der allmählichen Prothesen-Degeneration gerade bei aus Gewebe gewonnenen bioprosthetischen Herzklappen von besonderer klinischer Bedeutung. Dennoch ist die Pathogenese der Verkalkung nicht vollständig aufgeklärt und darüber hinaus gibt es hierfür auch heute noch keine ausreichend wirksame Therapie.

Ohne auf eine bestimmte Theorie zu favorisieren bzw. darauf festgelegt zu werden, sollen hier mögliche Ursachen kurz beschrieben werden. Als ein Ursprung der Mineralisierung und insbesondere der Verkalkung wurde z.B. nachgewiesen, dass diese in erster Linie bei den Zelltrümmern beginnt, die in Gewebematrizen von bioprothetischen Herzklappen und zwar gleichermaßen bei bioprothetischen Herzklappen mit Herkunft aus Perikard oder Aortenwurzel, auftreten. Die Verkalkung von bioprothetischem vernetztem Gewebe wurde auch auf die Gegenwart von alkalischer Phosphatase in den Zelltrümmern und deren mögliche Akkumulation innerhalb des implantierten Gewebes aus dem Blut, in Verbindung gebracht. Die Mineralisierung könnte auch dadurch bedingt sein, dass Phospholipide in den Zelltrümmern Kalzium sequestrieren und die Keimstelle von Apatit (Calciumphosphat) bilden. Weiterhin wurde vorgeschlagen, dass Untereinheiten von Elastin und Fibrillin wegen der Calcium-bindenden Funktion dieser Proteine eine Ursache für die Verkalkung sein können. Unabhängig von ihrem Mechanismus wird die Mineralisierung in Bioprothesen und insbesondere die Verkalkung als die häufigste Ursache des klinischen Versagens von bioprothetischen Herzklappen angesehen, die aus Schweine-Aortenklappen oder Rinderperikard gewonnen werden. Bei menschlichen Aorten-homograft-Implantaten wurde die pathologische Verkalkung, auch wenn sie sich hier langsamer als bei bioprothetischen Herzklappen vollzieht, ebenfalls beobachtet und sie betrifft hier sowohl die Klappengewebe wie auch die benachbarte Aorten-Wand. Pathologische Verkalkung führt letztlich zum Klappenversagen, z.B. in Form von Stenose und/oder Regeneration, und erfordert eine Re-Implantation. Da bioprothetische Herzklappen und auch Homograft-Herzklappen der Verkalkung unterliegen, ist deren klinische Verwendung, trotz einiger Lösungsansätze, die Mineralisierung bzw. die Verkalkung zu verringern oder zu verhindern, heute noch beschränkt.

Im Stand der Technik werden mehrere Methoden angewandt, um die Mineralisierung bzw. die Verkalkung bei bioprothetischen Herzklappen soweit wie möglich zu verringern oder zu verhindern. In diesen Verfahren werden die bioprothetischen Herzklappen üblicherweise vor der Implantation mit verschiedenen Substanzen behandelt. Als geeignete Substanzen werden sulfatierte aliphatische Alkohole, Phosphatester, Aminosäuren, Diphosphonate, Derivate von Carbonsäuren und verschiedene Tenside beschrieben. Ein anderes Verfahren verwendet Amino-Ölsäure (AOA, amino oleic acid) als Mittel, um die Verkalkung in bioprothetischen Herzklappen aus Schweine-Aortenwurzel-Gewebe zu mildem. Eine wirksame Verhinderung der Mineralisierung der Aortenwand konnte durch Anwendung dieser Verfahren bisher jedoch nicht erzielt werden und eine erfolgreiche Lösung des Problems der nach der Implantation auftretenden Mineralisierung steht noch aus.

Weiterhin können Bioprothesen aus tierischen Geweben, z.B. Schweine-Herzklappen, unterschiedlich starke immunogene und entzündliche Reaktionen beim Empfänger auslösen. Im Allgemeinen wird daher auch hier versucht, durch chemische Behandlung der tierischen Gewebe eine immunologische Abstoßung zu verhindern. Zwar kann die gegenwärtige Methode der Glutaraldehyd-Fixierung die Antigenität eines implantierten Schweine-Klappen-Gewebes deutlich maskieren, aber nicht völlig beseitigen. Schweine-Herzklappen können daher selbst nach einer Glutaraldehyd-Fixierung noch zu einer leichten bis schweren entzündlichen Reaktion führen, die zum Teil auf die zytotoxische Natur von Glutaraldehyd zurückgeführt werden kann, und in schweren Fällen kann das fremde Gewebe sogar eine chronische entzündliche Reaktion verursachen.

Als beispielhafte Methode zur Behandlung von Geweben vor einer Transplantation sei auf die Patentveröffentlichungen US 6,166,184 (Methods for making bioprosthetic devices) und US 6,509,145 (Process for reducing mineralization of tissue used in transplantation), und die veröffentlichten US-Patentanmeldungen US 7,078,163, US 2003/0118981 A1 (Process for reducing mineralization of tissue used in transplantation), WO 2005/011764, US 2005/0020506 A1 (Crosslinked compositions comprising collagen and demineralized bone matrix, methods of making and methods of use) verwiesen, die Verfahren für die Behandlung von biologischem Gewebe vor einer Implantation beschreiben.

Bisher werden die biologischen Herzklappen, z.B. die Herzklappensegel, auch dezellularisiert. Unter Dezellularisierung versteht man Verfahren zur Entfernung von Zellen und Zelltrümmern aus Gewebe und Gewebekonstrukten. Diese Dezellularisierung wird in der Regel bei aus Schweine-Aortenklappen oder Rinderperikard hergestellten bioprothetischen Herzklappen angewandt.

So beschreibt die US 2005/0266390 A1 ein Verfahren zur Dezellularisierung von Säugetiergewebe für den Einsatz in der Transplantationsmedizin und Tissue Engineering. Bei diesem Verfahren wird ein ionisches Detergens und ein nichtionisches Detergens für einen längeren Zeitraum, der mehr als fünf Tage betragen kann, gleichzeitig zur Anwendung am Säugetiergewebe gebracht. Hierbei wird z.B. SDS als ionisches Detergens und Triton X-100 als nicht-ionisches Detergens verwendet. Es folgt ein ausgedehnter Spülschritt, der ebenfalls mehr als fünf Tage dauern kann. Diese Extraktion mit nachfolgendem Spülen soll ein Gewebe mit Spannungs-Dehnungs-Kurven und DSC-Daten ähnlich wie bei frischen, unverarbeiteten Geweben liefern. Die US 2005/0266390 A1 gibt auch an, dass das verarbeitete Gewebe größtenteils frei von Zellen ist, die zugrundeliegende Struktur im Wesentlichen intakt bleibt und diese, auch ohne Fixierung mit Glutaraldehyd, ein deutlich verbessertes Verhalten hinsichtlich Entzündungsreaktionen relativ zu frischem Gewebe zeigt, sowie eine deutlich geringere Verkalkung *in situ* bezogen auf Glutaraldehyd-fixierte Gewebe aufweist. Das Verfahren kann z.B. für die Dezellularisierung von Schweineherz-Klappensegel und Schweineherz-Wandgewebe vor Verwendung bei der Transplantation eingesetzt werden.

Auch die WO 2005/118014 beschreibt ein Verfahren zur Dezellularisierung für Gewebe, das als bioprosthetischer Ersatz bei Transplantationen verwendet werden sollen. Die Methode der WO 2005/118014 betrifft die Behandlung von aus einem Tier ausgeschnittenem Gewebe, das noch Zellmembranen enthält, für die Herstellung einer Gewebe-basierten implantierbaren Bioprothese. Bei diesem Verfahren wird das ausgeschnittene Gewebe gleichzeitig mit zwei unterschiedlichen Detergenzien kontaktiert, wobei das eine ein ionisches Detergens ist, das Zellmembranen zerstören kann, und das andere ein Detergens mit einer neutralen Netto-Ladung ist.

Die Dezellularisierung führt jedoch zu einem Material, das mechanisch unzureichende Eigenschaften aufweist. Um die mechanischen Eigenschaften des Materials zu verbessern wird im Stand der Technik das gesamte Material, d.h. durch Behandlung der gesamten Oberfläche und der darunter liegenden Schichten, chemisch vernetzt und so mechanisch verbessert. Die chemische Vernetzung birgt jedoch Risiken. So müssen nicht abreagierte Gruppen aufwändig entfernt werden, um eine ausreichende Biokompatibilität des Materials zu gewährleisten. Als Beispiele für diese Bemühungen im Stand der Technik, Vernetzungsreagentien wie Glutaraldehyd wieder zu entfernen seien die Verfahren "ThermaFix" (Edwards) oder das "AOA Tissue Treatment" (Medtronic) genannt. Beim "AOA Tissue Treatment" handelt es sich z.B. um die oben bereits erwähnte Behandlung des Gewebes mit Amino-Ölsäure (AOA, amino oleic acid).

Beim "ThermaFix"-Prozess handelt es sich um eine gegen Kalkablagerungen wirkende Gewebebehandlung. Die Glutaraldehydfixierung ist hierbei nur der erste Schritt der Gewebebehandlung (Southem LJ, et al. Glutaraldehyde - indeed cross-links: a study of model compounds and commercial bioprosthetic valves. J Heart Valve Dis 2000;9(2):241-8). Die Glutaraldehydfixierung unterstützt die Gewebekonservierung und Sterilität und verbessert die Biokompatibilität und strukturelle Stabilität. Werden bei Gewebebehandlungen im Stand der Technik Kalziumablagerungsstellen wie Phospholipide und instabile Glutaraldehydrestmoleküle nur chemisch gebunden, kann die Wirkung gegen Kalkablagerung mit der Zeit nachlassen. Gegenüber der alleinigen chemischen Bindung (Fixierung) kann durch eine nachträgliche Extraktion eine weitere Verbesserung erzielt werden. Beim erweiterten "ThermaFix" Gewebeverfahren werden daher zusätzlich noch die Kalziumablagerungsstellen wie Phospholipide und instabile Glutaraldehydrestmoleküle extrahiert.

In PAÉZ, J.M. GARCÌA et al. : "Resistance and Stability of A New Method for Bonding Biological Materials Using Sutures and Biological Adhesives", JOURNAL OF BIOMATERIALS APPLICATIONS, Band 19 - Januar 2005, Seiten 215-236, wird ein Verfahren zur Erhöhung der Widerstandskraft und Stabilität von bioprosthetischen Materialien, insbesondere Herzklappensegeln, beschrieben. Ausgewählte mechanisch beanspruchte Bereiche wie Kanten der Nähte werden chemisch stabilisiert durch Behandlung mit gepufferter Glutaraldehydlösung , In PAÉZ, J.M. GARCÌA et al. : "Mechanical Resistance of a New Biomaterial, Ostrich Pericardium, and a New Method of Joining Tissues Combining Suturing and a Biological Adhesive", JOURNAL OF BIOMEDICAL Materials Research 2004, Seiten 9-16, wird zusätzlich auf den Vorteil einer speziellen Nähtechnik hingewiesen.

US2002/0086977 offenbart ein Verfahren zur Aufbereitung eines hydrophilen, porösen Polymermaterials, bei dem zur Quervemetzung Glutaraldehy in Aceton eingesetzt wird. JONES J.G. et al : "The quantification of zinc in the mucosal cells of human small intestine using x-ray microanalysis " SCANDINAVIAN JOURNAL OF GASTROENTEROLOGY, SUPPLEMENT 1981, Band 70, 1981, Seiten 37-48, offenbart Untersuchungen zur Bestimmung des Zinkgehalts in intestinalen Mucosalzellen mittels Röntgenanalyse, wobei chirurgisch entnommenes Gewebe mit Glutaraldehyd und Aceton fixiert wird. US2003/028247 beschreibt ein Verfahren zum Zuschneiden von biologischem Material, z.B. für Herzklappenprothesen, mittels Laser. Dabei ist ein zu hoher Wärmeeintrag zu vermeiden, da sich verbranntes Gewebe zu sehr versteift.,

In DE 2715466 A1 ist ein Verfahren zur Herstellung von stabileren Materialien für biologische Implantate offenbart, bei dem das biologische Material mit einem Polymer durchtränkt und anschliessend in eine Glutaraldehydlösung gelegt wird. WO 01/28604 A1 offenbart die Präparation von biologischem Material für eine Herzklappenprothese, bei dem das implantierte Gewebe nach einer Vernetzung mit Glutaraldehydlösung später eine deutlich geringere Kalzifizierung aufweist. WO 01/97874 A1 beschreibt ebenfalls ein Verfahren zur Behandlung von biologischem Gewebe, bei dem für die Vernetzung Aldehyde, Isocyanate und Epoxyverbindungen in Frage kommen.

Im Abstract von VASUDEV; S.C. et al: "The anticalcification effect of polyethylene glycol-immobilized on hexamethylene diisocyanate treated pericardium ", ARTIFICIAL CELLS, BLOOD SUBSTITUITES, AND IMMOBILIZATION BIOTECHNOLOGY, Jan 2000, Band 28, Seiten 79-94, ist eine zu Glutaraldehyd alternative Vemetzungsmethode beschrieben, bei der hexamethylen diisocyanate als Vernetzer verwendet wird. WO 2006/066327 A1 zeigt Vergleichsdaten einer zusätzlichen Alkoholbehandlung zu einer reinen Glutaraldehydbehandlung auf.

Die Verfahren des Standes der Technik weisen den Nachteil auf, dass die gesamte Oberfläche des biologischen Materials chemisch, ggf. einschließlich teilweise oder vollständig der darunter liegenden Schichten, verändert wird, obwohl es in der Praxis hauptsächlich an den Rändern des biologischen Materials zu Rissbildungen und einem Versagen der Struktur kommt. Zusätzlich wird durch die Behandlung der kompletten Herzklappe das biologische Material insgesamt steifer und führt zu einem homogenen Material über die gesamte Herzklappe. Diese steht im Widerspruch zu den örtlich unterschiedlichen Anforderungen an die Herzklappe. So werden z.B. die Randbereiche an der Nahtstelle einer Herzklappe anders belastet, als die Randbereiche im Lumen, wobei die Anforderungen im Mittelbereich der Herzklappe wiederum ganz andere sind.

Im Stand der Technik besteht deshalb für Implantate immer noch ein großes Bedürfnis zur Verbesserung der Eigenschaften von bioprosthetischen Komponenten, die auf biologischen Materialien basieren oder diese umfassen, besonders im Hinblick auf z.B. mechanische Stabilität, Lebensdauer sowie Biokompatibilität. Ein besonderes Bedürfnis besteht hierbei für die Verbesserung der Eigenschaften von u.a. biologischen Herzklappen, insbesondere von biologischen Herzklappensegeln, für deren Verwendung als Implantat.

Es besteht daher nach wie vor ein hoher Bedarf an einem verbesserten bioprosthetischen bzw. biologischen Herzklappenersatz mit guter Hämodynamik, einer hohen Lebensdauer, und/oder verbesserter Biokompatibilität, z.B. mit einer ausreichenden Verminderung des Risikos der Induktion von Blutgerinnung, so dass nach der Implantation bevorzugt auf Antikoagulantien verzichtet werden kann, und/oder einem möglichst niedrigen Risiko der Mineralisierung und/oder Verkalkung. Es besteht auch ein Bedarf an anderen verbesserten, dauerhaften und nicht - oder gering -immunogenen, und hinsichtlich Heilung und Wachstum bei Patienten verträglichen bioprosthetischen Geweben.

Dementsprechend besteht auch ein Bedarf für die Bereitstellung von bioprothetischen (biologischen) Herzklappen und/oder von anderen, aus (homogenen oder xenogenen) Geweben gewonnenen Implantaten (bioprosthetischen Materialien) mit verbesserter Biokompatibilität, z.B. solche die in vivo einer pathologischen Verkalkung langfristig widerstehen, und wobei die bioprothetischen (biologischen) Implantate, insbesondere Herzklappen bevorzugt auch eine verbesserte langfristige mechanische Stabilität bzw. Dauerbelastbarkeit aufweisen. Auch gibt es einen Bedarf an Methoden, mit denen bioprothetische (homogenene oder xenogene) Gewebe (bzw. darauf basierende Implantate) mit verbesserter Biokompatibilität, z.B. solche mit verminderter Entzündungs- und Immunreaktion, bereitgestellt werden können, und durch die insbesondere auch bioprothetische (homogenen oder xenogene) Gewebe (bzw. darauf basierende Implantate) mit einer verbesserten langfristigen mechanischen Stabilität bzw. Dauerbelastbarkeit erzielt werden können.

Der vorliegenden Erfindung liegt daher die Aufgabe zugrunde, bioprosthetische Komponenten für Implantate, vorzugsweise bioprothetische (biologische) Herzklappen, insbesondere bioprothetische (biologische) Herzklappensegel, und gegebenenfalls auch andere aus (homogenen oder xenogenen) Geweben gewonnene Implantate mit verbesserter Biokompatibilität und insbesondere mit langfristiger mechanischer Stabilität bzw. Dauerbelastbarkeit bereitzustellen. Speziell liegt der Erfindung in einer bevorzugten Variante die Aufgabe zugrunde, bioprothetische (biologische) Herzklappen, insbesondere bioprothetische (biologische) Herzklappensegel, mit verbesserter Biokompatibilität, z.B. solche die in vivo einer pathologischen Verkalkung möglichst langfristig widerstehen, und insbesondere eine verbesserte langfristige mechanische Stabilität bzw. Dauerbelastbarkeit aufweisen, bereitzustellen.

In einer anderen Variante liegt der Erfindung die Aufgabe zugrunde, auch andere aus (homogenen oder xenogenen) Geweben gewonnene Implantate (bioprosthetische Materialien) mit verbesserter Biokompatibilität z.B. solche die in vivo einer pathologischen Verkalkung möglichst langfristig widerstehen, und insbesondere solche mit einer verbesserten langfristigen mechanischen Stabilität bzw. Dauerbelastbarkeit, bereitzustellen.

Eine weitere Aufgabe besteht darin, auch Methoden anzugeben, mit denen bioprothetische (biologische) Herzklappen mit den vorstehenden verbesserten Eigenschaften, insbesondere bioprothetische (biologische) Herzklappensegel, und gegebenenfalls auch anderen aus (homogenen oder xenogenen) Geweben gewonnene Implantate (bioprosthetische Materialien) mit verbesserter Biokompatibilität und insbesondere mit einer langfristigen mechanischen Stabilität bzw. Dauerbelastbarkeit hergestellt werden können.

Die Aufgabe wird gelöst, wie nachfolgend noch detaillierter beschrieben, indem eine nur (flächenbezogene) partielle stabilisierende Behandlung des ausgewählten biologischen Gewebes erfolgt, wobei durch die partielle Behandlung bevorzugt eine mechanische Stabilität genau dort erzeugt wird, wo es bei Dauerbelastung auch zum Versagen des Materials kommen kann. Gleichzeitig ist beim ausgewählten biologischen Gewebe der Umfang der Modifikationdurch chemische Substanzen örtlich begrenzt. Der Großteil der Oberfläche des ausgewählten biologischen Gewebes bleibt gemäß der vorliegenden Erfindung nativ unbehandelt oder wenigstens durch die (zusätzliche) stabilisierende Behandlung unberührt und damit auch biokompatibler als das vollständig behandelte biologische Gewebe. Die vorliegende Erfindung findet hierbei insbesondere, jedoch nicht einschränkend, Anwendung bei der Verwendung von Säugetiergewebe, z.B. Schweine-Aortenklappen oder Rinderperikard, für die Herstellung von bioprothetischen (biologischen) Herzklappen, z.B. vorzugsweise für die Herstellung von bioprothetischen (biologischen) Herzklappensegeln.

Die vorliegende Erfindung wird weiter unten zwar, ohne sie hierdurch einschränken zu wollen, am Beispiel von biologischen Herzklappen, insbesondere biologischen Herzklappensegeln näher beschrieben, sie kann analog viel allgemeiner aber auch auf andere biologische oder bioprosthetische Gewebe angewandt werden, und somit auch bei diesen anderen Bioprothesen eine bedeutende Rolle spielen. Neben den bereits angesprochenen Herzklappen, können solche Bioprothesen z.B. auch Gefäßprothesen, Venentransplantate, oder Harnblasen, Herz-Blasen, linksventrikuläre Hilfsmittel und dergleichen sein, die unter Verwendung natürlicher, z.B. auch homogener oder xenogener, Gewebe gewonnen werden.

Die vorliegende Erfindung betrifft daher in ihrer breitesten Ausgestaltung generell bioprosthetische Komponenten für Implantate, vorzugsweise bioprothetische (biologische) Herzklappen, insbesondere bioprothetische (biologische) Herzklappensegel, und gegebenenfalls auch andere aus (homogenen oder xenogenen) Geweben gewonnene Implantate mit verbesserter Biokompatibilität und insbesondere mit langfristiger mechanischer Stabilität bzw. Dauerbelastbarkeit. Die Erfindung ist anwendbar auf alle Arten von Implantaten bei dehnen Gewebe zum Einsatz kommt, wie z.B. Grafts wie Closure Devices, allgemein bei Klappensystemen wie Venenklappen oder Herzklappen, wie z.B. Herzsegel, Mitralklappen, Aortenklappen oder Pulmonarklappen. Bei den sog. Closure Divices kann die vorliegende Erfindung auf Implantate oder biologischen Komponenten davon, z.B. im Hirnbereich (bei Hirn-Aneurysma) oder im Blutgefäßsystem, insbesondere dem Herzen, bei dort vorkommenden Aneurysmen angewendet werden. Sie kann auch auf Herzsegel zum Abdichten einer Narbe angewendet werden, wobei die Narbe z.B. durch eine Intervention oder eine Behandlung einer genetisch bedingten Öffnung zwischen Herzkompartimenten hervorgerufen sein kann. Beispiele für solche durch einen Gendefekt hervorgerufene Öffnung, welche häufig schon im Kindesalter behandlungspflichtig ist, sind der Atrio-ventrikuläre Septumdefekt, Atriumseptumdefekt und der Ventrikelseptumdefekt.

Die Erfindung betrifft somit generell eine bioprosthetische Komponente für ein Implantat, basierend auf einem nativen oder ggf. bereits modifizierten biologischen Material, worin die Oberfläche des biologischen Materials aufgrund einer gesonderten stabilitätssteigernden Behandlung nur eines Teils der Oberfläche eine oder mehrere Zonen mit einer, im Vergleich zur mechanischen Stabilität vor der stabilitätssteigernden Behandlung, gesteigerten mechanischen Stabilität aufweist, und wobei, jeweils bezogen auf die gesamte Oberfläche der bioprosthetischen Komponente als 100%, die Zonen mit gesteigerter mechanischer Stabilität einen Anteil ≤ 40% der Oberfläche ausmachen und die Eigenschaften der übrigen Zonen mit einem Anteil von ≥ 60% der Oberfläche vor und nach der stabilitätssteigernden Behandlung im Wesentlichen unverändert sind.

Bevorzugt ist, dass ≥ 0,5 %, bevorzugt ≥ 2 %, weiter bevorzugt ≥ 5 % und besonders bevorzugt ≥ 10 % der Oberfläche der bioprosthetischen Komponente durch die (flächenmäßig begrenzte) Oberflächenbehandlung modifiziert ist. Entsprechend beträgt dann der Anteil der vor und nach der stabilitätssteigernden Behandlung im Wesentlichen unveränderten Oberfläche der bioprosthetischen Komponente ≤ 99,5 %, bevorzugt ≤ 98 %, weiter bevorzugt ≤ 95 %, und besonders bevorzugt ≤ 90v% der Oberfläche.

Die erfindungsgemäße bioprosthetische Komponente für ein Implantat kann hierbei für ein Implantat bestimmt sein, das ausgewählt ist aus der Gruppe bestehend aus (1) Closure Devices, vorzugsweise Aneurysmen-Stents für den gesamten Blutkreislauf einschließlich des Blutkreislaufs im Hirnbereich; (2) Klappensystemen, vorzugsweise biologische Herzklappen und Venenklappen, besonders bevorzugt biologische Herzklappen ausgewählt aus der Gruppe bestehend aus Herzklappensegeln, Mitral-Herzklappen, Aorten-Herzklappen oder Pulmonar-Herzklappen; und (3) Gewebesegeln zum Abdichten von Organnarben, vorzugsweise Herzsegel zum Abdichten von Narben am Herzen, besonders bevorzugt Herzsegel zum Abdichten von Narben am Herzen bei einem atrio-ventrikulären Septumdefekt, einem Atriumseptumsefekt oder Ventrikelseptumdefekt.

Eine solche erfindungsgemäße bioprosthetische Komponente für ein Implantat kann sich weiterhin dadurch auszeichnen, dass die Oberfläche der bioprosthetischen Komponente eine oder mehrere Zonen mit gesteigerter mechanischer Stabilität aufweist, wobei die gesteigerte mechanische Stabilität auf einer gesonderten stabilitätssteigernden Behandlung der Oberfläche dieser Zonen beruht, die eine stabilitätssteigernde chemische Behandlung ist. Beispiele für solche gesonderte stabilitätssteigernde Behandlungen der Oberfläche bzw. der genannten Zonen der Oberfläche sind weiter unten im Zusammenhang mit Herzklappen, insbesondere Herzsegeln, detaillierter beschrieben.

In vorteilhaften erfindungsgemäßen bioprosthetischen Komponenten für Implantate weist die Oberfläche der bioprosthetischen Komponente eine oder mehrere Zonen mit gesteigerter mechanischer Stabilität auf, wobei eine solche Zone bei zweckentsprechender Verwendung der bioprosthetischen Komponente einer erhöhten mechanischen Dauerbelastung unterliegt, und wobei vorzugsweise diese Zone wenigstens eine Kante und/oder Nahtrand und/oder Nahtbereich und/oder sonstiger Fixierungsbereich der bioprosthetischen Komponente oder eine Kombination dieser Zonen ist, und wobei besonders bevorzugt diese Zone wenigstens ein Nahtrand oder Nahtbereich der bioprosthetischen Komponente ist.

Wie vorstehend bereits angegeben, ist bei einer erfindungsgemäßen bioprosthetischen Komponente für ein Implantat zweckmäßiger Weise nicht die gesamte Oberfläche des biologischen Materials durch eine gesonderte zusätzliche stabilitätssteigernde Behandlung verändert bzw. stabilisiert. Eine zweckmäßige Variante der Erfindung betrifft daher eine bioprosthetische Komponente für ein Implantat, die sich dadurch auszeichnet, dass darin die Zonen mit gesteigerter mechanischer Stabilität, jeweils bezogen auf die gesamte Oberfläche der bioprosthetischen Komponente als 100%, einen Anteil von ≤ 30%, insbesondere von ≤ 20%, vorzugsweise einen Anteil von 5 bis 20%, besonders bevorzugt einen Anteil von 10 bis 20%, der Oberfläche ausmachen. Eine weitere zweckmäßige Variante der Erfindung kann daher auch eine bioprosthetische Komponente für ein Implantat sein, die sich dadurch auszeichnet, dass darin die Eigenschaften der übrigen Zonen, d.h. der Zonen ohne eine gesonderte zusätzliche stabilitätssteigernde Behandlung, jeweils bezogen auf die gesamte Oberfläche der der bioprosthetischen Komponente als 100%, mit einem Anteil von ≥ 70%, insbesondere ≥ 80%, vorzugsweise von 95 bis 80%, besonders von 90 bis 80%, der Oberfläche des Herzklappensegels vor und nach der stabilitätssteigernden Behandlung im Wesentlichen unverändert sind.

Die Erfindung wird im Folgenden zunächst allgemein für bioprosthetische Komponenten für Implantate weiter beschrieben. Nähere Einzelheiten für die nachfolgenden allgemeinen Ausgestaltungen der Erfindung für bioprosthetische Komponenten können den weiter unten am Beispiel von Herzklappen, insbesondere am Beispiel Herzklappensegeln, detaillierter beschriebenen Ausführungen der Erfindung entnommen und analog oder durch den Fachmann gewünschtenfalls in angepasster Weise angewandt werden.

In den bioprosthetischen Komponenten für ein Implantat, basierend auf einem nativen oder ggf. bereits modifizierten biologischen Material, kann in einer weiteren Variante der Erfindung das biologische Material ein durch chemische Vorbehandlung bereits modifiziertes biologisches Material, vorzugsweise ein durch Dezellularisierung modifiziertes biologisches Material, sein.

Eine erfindungsgemäße bioprosthetische Komponente für ein Implantat zeichnet sich dadurch aus, dass das Implantat eine biologische Herzklappe, insbesondere ein Herzklappensegel, basierend auf einem nativen oder ggf. bereits modifizierten biologischen Material, ist. Sehr bevorzugt ist hierbei eine biologische Herzklappe, insbesondere ein Herzklappensegel, basierend auf einem nativen oder ggf. bereits modifizierten biologischen Material, worin das biologische Material ein durch chemische Vorbehandlung bereits modifiziertes biologisches Material, besonders bevorzugt ein durch Dezellularisierung modifiziertes biologisches Material, ist.

Die Erfindung betrifft eine bioprosthetische Komponente für ein Implantat, die dadurch gekennzeichnet ist, dass die bioprosthetische Komponente, vorzugsweise eine biologische Herzklappe, insbesondere ein Herzklappensegel, auf einem biologischem Material basiert, und wobei die Oberfläche der bioprosthetischen Komponente, vorzugsweise der biologischen Herzklappe, insbesondere des Herzklappensegels, eine oder mehrere Zonen mit gesteigerter mechanischer Stabilität aufweist, wobei die gesteigerte mechanische Stabilität auf einer gesonderten stabilitätssteigernden Behandlung der Oberfläche dieser Zone mit Divinylsulfon und einer Mischung aus einem verzweigten Polyethylenamin und Divinylsulfon beruht.

Die erfindungsgemäße bioprosthetische Komponente für ein Implantat kann im Randbereich sehr vorteilhaft mit einer Naht versehen werden. Dabei kann der Randbereich mit jedem im Stand der Technik an sich bekannten, zum Vernähen von biologischen Materialien geeigneten Faden vernäht in an sich üblicher, dem Fachmann bekannter Weise vernäht werden. Verschiedene Nähtechniken sind aus der Textilindustrie bekannt und können angewendet werden. Als Nahtmaterial kann ein Monofilament oder ein Multifilament eingesetzt werden. Beispielsweise sind TEVDEK II^{®} von Deknatel oder PROFILEN^{®} von Lenzing gut geeignet. Pures Teflon hat sich aufgrund seines geringen Reibwertes beim Nähen besonders bewährt.

Bioprosthetische Komponenten für ein Implantat gemäß der Ausgestaltungen der Erfindung sind vorzugsweise weiterhin dadurch gekennzeichnet, dass die bioprosthetische Komponente, vorzugsweise eine biologische Herzklappe, insbesondere ein Herzklappensegel, auf einem biologischem Material basiert, und wobei die Oberfläche der bioprosthetischen Komponente, vorzugsweise der biologischen Herzklappe, insbesondere des Herzklappensegels, eine oder mehrere Zonen mit gesteigerter mechanischer Stabilität aufweist, wobei eine solche Zone folgende Materialeigenschaft und/oder Rissfestigkeit aufweist: SRS von 30 bis 450 N/mm², vorzugsweise von 50 bis 300 N/mm², weiter bevorzugt von 100 bis 200 N/mm², besonders bevorzugt von 150 bis 200 N/mm². Die Bestimmung der Nahtausreisfestigkeit von Probenmaterial kann in 0.9%-iger physiologischer Salzlösung bei 37°C mit Hilfe einer Prüfmaschine ermittelt werden, die in der Lage ist, eine kontinuierliche Prüfgeschwindigkeit (mm/min) aufrechtzuerhalten und den Kraft-Dehnungsverlauf zu ermitteln. Details zur Bestimmung der Nahtausreisfestigkeit von Probenmaterial sind weiter unten am Beispiel von Herzklappen näher beschrieben. Vorbeschrieben und auch nachfolgend werden die erfindungsgemäßen bioprosthetischen Komponenten bzw. Implantate teilweise durch ihr Herstellungsverfahren charakterisiert. Für den Fachmann ist dabei klar, dass es im Sinne der Erfindung nicht darauf ankommt, dass tatsächlich das charakterisierende Herstellungsverfahren für die jeweilige Komponente/das jeweilige Implantat angewendet wurde, sondern nur darauf, dass die erfindungsgemäße Komponente/das erfindungsgemäße Implantat mit Merkmalen bzw. Eigenschaften vorliegt, die auch mit dem zur Charakterisierung verwendeten Verfahren erreicht werden können. Dementsprechend sind auch Gegenstände von der Erfindung erfasst, die tatsächlich nicht mit dem zur Charakterisierung beschriebenen Verfahren hergestellt worden sind, mit diesem aber hätten hergestellt werden können.

Schließlich betrifft die Erfindung auch ein Verfahren zur Herstellung einer bioprosthetischen Komponente für ein Implantat, vorzugsweise einer biologischen Herzklappe, insbesondere eines Herzklappensegels, wie gemäß der vorliegenden Erfindung definiert, wobei die bioprosthetische Komponente für ein Implantat, vorzugsweise die biologische Herzklappe, insbesondere das Herzklappensegel, auf einem nativen oder ggf. bereits modifizierten biologischen Material basiert und wobei die Oberfläche der bioprosthetischen Komponente für ein Implantat, vorzugsweise der biologische Herzklappe, insbesondere des Herzklappensegels, eine oder mehrere Zonen mit einer gesteigerten mechanischen Stabilität aufweist, wobei sich das erfindungsgemäße Verfahren dadurch auszeichnet, dass man nur einen Teil der Oberfläche der bioprosthetischen Komponente für ein Implantat, vorzugsweise der biologischen Herzklappe, insbesondere des Herzklappensegels, einer gesonderten stabilitätssteigernden chemischen Behandlung der Oberfläche dieser Zone mit Divinylsulfon und einer Mischung aus einem verzweigten Polyethylenamin und Divinylsulfon unterwirft, um eine oder mehrere Zonen mit einer, im Vergleich zur mechanischen Stabilität vor der stabilitätssteigernden Behandlung, gesteigerten mechanischen Stabilität zu erzeugen, und wobei, jeweils bezogen auf die gesamte Oberfläche der bioprosthetischen Komponente für ein Implantat bzw. vorzugsweise der biologischen Herzklappe bzw. insbesondere des Herzklappensegels als 100%, die Zonen mit gesteigerter mechanischer Stabilität mit maximal einem Anteil von bis zu 40% der Oberfläche gebildet werden und die Eigenschaften der übrigen Zonen mit einem Anteil von wenigstens 60% der Oberfläche durch die stabilitätssteigernde Behandlung im Wesentlichen nicht verändert werden.

Speziell stellt die Erfindung zur Lösung der ihr besonders zugrundeliegenden Aufgabe, bioprothetische (biologische) Herzklappen, insbesondere bioprothetische (biologische) Herzklappensegel, mit verbesserter Biokompatibilität, z.B. solche die in vivo einer pathologischen Verkalkung möglichst langfristig widerstehen, und insbesondere eine verbesserte langfristige mechanische Stabilität bzw. Dauerbelastbarkeit aufweisen, bereit.

Die Erfindung soll daher nun am Beispiel von biologischen Herzklappen näher ausgeführt werden. Die Erfindung betrifft diesbezüglich in einer Ausgestaltung eine biologische Herzklappe, insbesondere ein Herzklappensegel, basierend auf einem nativen oder ggf. bereits modifizierten biologischen Material, dessen Oberfläche aufgrund einer gesonderten stabilitätssteigernden Behandlung nur eines Teils der Oberfläche eine oder mehrere Zonen mit einer, im Vergleich zur mechanischen Stabilität vor der stabilitätssteigernden Behandlung, gesteigerten mechanischen Stabilität aufweist, und wobei, jeweils bezogen auf die gesamte Oberfläche der biologischen Herzklappe bzw. insbesondere des Herzklappensegels als 100%, die Zonen mit gesteigerter mechanischer Stabilität maximal einen Anteil von ≤ 40% der Oberfläche ausmachen und die Eigenschaften der übrigen Zonen mit einem Anteil von ≥ 60% der Oberfläche vor und nach der stabilitätssteigernden Behandlung im Wesentlichen unverändert sind.

Die Erfindung kann auf alle Arten von biologischen Herzklappen angewandt werden. Solche biologischen Herzklappen können aus Schweinen oder aus anderen oder unter Verwendung von anderen tierischen oder menschlichen Geweben gewonnen werden. Bei diesen biologischen Herzklappen kann das Klappengewebe aus menschlichem (Homograft) oder tierischem (Xenograft) Gewebe bestehen. Insbesondere können Schweine-Aortenklappen oder Rinderperikard als Grundlage für die biologischen Herzklappen, insbesondere die biologischen Herzklappensegel, dienen. Tier- und menschliche Spenderklappen müssen nach der Entnahme für die spätere Implantierung konserviert werden. Hierbei hat sich die Kryokonservierung in flüssigem Stickstoff als effektivstes Verfahren durchgesetzt. Alternativen sind die Präparation in einer antibiotischen Lösung bei 4°C, Röntgenbestrahlung und Trockengefrierung.

So kann durch die Erfindung biologischer Ersatz für alle der insgesamt vier natürlichen Herzklappen bereitgestellt werden. Die natürlichen Herzklappen wirken im Herz als Ventile und verhindern einen Rückstrom des Blutes in die falsche Richtung. Sie werden gebildet von segelförmigen oder taschenförmigen, in die Lichtung vorragenden Gebilden, welche Duplikaturen des Endokards sind, die innen durch Bindegewebe verstärkt sind. Jede Herzhälfte hat eine Segelklappe (Atrioventrikularklappe) und eine Taschenklappe (Semilunarklappe). Die Segelklappen liegen zwischen Vorhof und Kammer und heißen Bikuspidalklappe oder Mitralklappe (links) und Trikuspidalklappe (rechts). Die Taschenklappen liegen jeweils zwischen Kammer und Ausstromgefäß und heißen Pulmonalklappe (rechts) und Aortenklappe (links).

Die natürlichen Herzklappen verwenden Flügelblätter aus dünnem, flexiblem Gewebe als Verschlusselement. Die Flügelblätter bewegen sich leicht aus der Öffnung, sobald das Blut durch die Herzklappe zu fließen beginnt, so dass die Strömung des Blutes durch die geöffnete Herzklappe durch die Flügelblätter nicht behindert wird. Die erfindungsgemäßen biologischen Herzklappen funktionieren analog und ermöglichen so eine relativ uneingeschränkte Durchflussöffnung, wenn die Herzklappe geöffnet ist.

Als Segelklappe werden diejenigen Herzklappen bezeichnet, die durch segelartige Zipfel (Cuspes) gekennzeichnet sind. Segelklappen finden sich am Herz der Säugetiere beidseits zwischen dem Vorhof (Atrium) und der Herzkammer (Ventriculus) und werden deshalb auch Atrioventrikularklappen (AV-Klappen, Valvae atrioventriculares) genannt. Die Segelklappe der rechten Herzseite besteht bei Säugetieren aus drei Segeln und wird deshalb als Trikuspidalklappe bezeichnet. Die Segelklappe der linken Herzseite hat bei Säugetieren nur zwei Segel und wird deshalb Bikuspidalklappe genannt. Die Segel sind an ihrer Basis am Herzskelett, an ihrem freien Rand über Chordae tendineae über die Papillarmuskeln befestigt.

Histologisch bestehen Segelklappen aus vier Schichten. Die dem Herzvorhof zugewandte Vorhofschicht (Lamina atrialis) besteht aus einer Lage Endothelzellen, die sich aus dem Endokard ableiten. Sie sind von einer dünnen Schicht Bindegewebsfasern und glatten Muskelzellen unterlagert. Unter der Atrialis liegt die Schwammschicht (Lamina spongiosa) aus lockerem Bindegewebe mit Kollagenfasern, Fibroblasten, elastischen Fasern sowie Anitschkow-Zellen, die in eine Grundsubstanz aus Proteoglykanen eingebettet sind. Die Bindegewebsschicht (Lamina fibrosa) besteht aus dichtem Bindegewebe und setzt sich an der Klappenbasis in die Anuli fibrosi des Herzskeletts, an den freien Rändern in die Eigenschicht der Sehnenfäden fort. Die der Herzkammer zugewandte Kammerschicht (Lamina ventriclaris) besteht wie die Atrialis aus Endothelzellen und Bindegewebe, allerdings sind hier keine glatten Muskelzellen eingelagert.

Als Taschenklappen oder Semilunarklappe bezeichnet man diejenigen Herzklappen, die durch halbmondförmige, schwalbennesterartig angeordneten Taschen (Valvulae semilunares) gekennzeichnet sind. Taschenklappen finden sich am Herz der Säugetiere an den beiden Ausstrombahnen der Herzkammern. Die Taschenklappe der Aorta wird als Aortenklappe (Valva aortae), die des Truncus pulmonalis Pulmonalklappe (Valva trunci pulmonalis) bezeichnet. Taschenklappen bestehen aus einer Duplikatur der Herzinnenhaut (Endokard). Die freien Ränder können knötchenartige Verdickungen aufweisen, die als Noduli valvarum semilunarium bezeichnet werden und den Klappenschluss verbessern. Durch die Erfindung werden wesentliche Vorteile gegenüber dem Stand der Technik bei bioprosthetischen Materialien, insbesondere bei biologischen Herzklappen und speziell Herzklappensegeln, erzielt. So wird das bioprosthetische bzw. biologische Material gemäß der vorliegenden Erfindung nur partiell mechanisch stabilisiert. Die gesonderte stabilitätssteigernde Behandlung des (ausgewählten) Teils der Oberfläche von bioprosthetischen bzw. biologischen Materialien, insbesondere von bioprosthetischen bzw. biologischen Herzklappen und speziell von bioprosthetischen bzw. biologischen Herzklappensegeln, besteht bevorzugt darin, dass durch chemische Behandlung eine Vernetzung und mechanische Stabilisierung erreicht wird. Demgemäß betrifft die Erfindung in einer Ausgestaltung eine biologische Herzklappe, insbesondere ein biologisches Herzklappensegel, basierend auf biologischem Material, dessen Oberfläche eine oder mehrere Zonen mit gesteigerter mechanischer Stabilität aufweist, wobei die gesteigerte mechanische Stabilität auf einer gesonderten stabilitätssteigernden Behandlung der Oberfläche dieser Zonen beruht, die eine gesonderte stabilitätssteigernde chemische Behandlung ist.

Gemäß der Erfindung wird besonderes Augenmerk auf die mechanische Stabilisierung von Randbereichen, insbesondere von mechanisch dauerhaft belasteten Randbereichen, gelegt. Diese Randbereiche erfahren aufgrund der gesonderten stabilitätssteigernden Behandlung nur dieses ausgewählten Teils der Oberfläche durch Vernetzung eine mechanische Stabilisierung. Neben den Randbereichen wie Kanten kann auch der Nähbereich des biologischen Materials zusätzlich stabilisiert werden. Gerade im Nähbereich sind die mechanischen Anforderungen ganz anders gelagert, als in den übrigen Bereichen des biologischen Materials, insbesondere einer biologischen Herzklappe und speziell eines Herzklappensegels. Der Nähbereich einer erfindungsgemäßen biologischen Herzklappe, insbesondere eines erfindungsgemäßen biologischen Herzklappensegels, zeichnet sich dadurch aus, dass ein Faden sich bei Zugbelastung nicht durch das Gewebe arbeitet und somit das Gewebe ausreißt. Durch die erfindungsgemäße Lösung sind bioprosthetische bzw. biologische Materialien, insbesondere bioprosthetische bzw. biologische Herzklappen und speziell bioprosthetische bzw. biologische Herzklappensegel verfügbar, die veränderte Materialeigenschaften in Abhängigkeit vom Ort aufweisen. Dieser Gradient der örtlich variierenden Materialeigenschaften hat direkten Einfluss auf die Rissfestigkeit und/oder andere Materialkennwerte.

Die Nahtausreisfestigkeit von Probenmaterial kann in 0.9%-iger physiologischer Salzlösung bei 37°C mit Hilfe einer Prüfmaschine ermittelt werden, die in der Lage ist, eine kontinuierliche Prüfgeschwindigkeit von ca. 10-100 mm/min aufrechtzuerhalten und den Kraft-Dehnungsverlauf zu ermitteln. Probenmaterial wird dazu mittig in einer Spannbacke positioniert und einseitig fixiert. Anschließend wird zwei Millimeter vom Rand der Probe (rechteckige Probengeometrie) eine Schlaufe aus chirurgischem Nahtmaterial angebracht und diese in eine zweite, obere Spannbacke gelegt und befestigt. Nach Fixierung der Probe erfolgt die Messung zur Ermittlung der Maximalkraft und maximalen Dehnung. Die Nahtausreißfestigkeit (SRS) in N/mm² wird aus der Maximalkraft, und der Proben- und Nahtmaterialdicke ermittelt. Die SRS z.B. für fixiertes biologisches Perikard zum Einsatz für Herzklappen liegt im Bereich von > 100 N/mm². Teilfixierte Bereiche der Klappensegel aus bioprosthetischen bzw. biologischen Materialien, und hier speziell biologische Herzklappen, insbesondere einem biologischen Herzklappensegel, basierend auf biologischem Material, dessen Oberfläche eine oder mehrere Zonen mit gesteigerter mechanischer Stabilität aufweist genügen hinsichtlich der Materialbelastbarkeit mindestens diesen Anforderungen an die SRS.

In einer Ausgestaltung betrifft die vorliegende Erfindung daher bioprosthetische bzw. biologische Materialien, und speziell eine biologische Herzklappe, insbesondere ein biologisches Herzklappensegel, basierend auf biologischem Material, dessen Oberfläche eine oder mehrere Zonen mit gesteigerter mechanischer Stabilität aufweist, wobei eine solche Zone bei zweckentsprechender Verwendung der Herzklappe, insbesondere des Herzklappensegels, einer erhöhten mechanischen Dauerbelastung unterliegt, und vorzugsweise wenigstens eine Kante und/oder Nahtrand und/oder Nahtbereich und/oder sonstiger Fixierungsbereich des Herzklappensegels oder eine Kombination davon, und besonders bevorzugt wenigstens ein Nahtrand oder Nahtbereich des Herzklappensegels, ist.

Der Anteil der Oberfläche, des bioprosthetischen bzw. biologischen Materials, und speziell der biologischen Herzklappe und insbesondere des biologischen Herzklappensegels, der gemäß der vorliegenden Erfindung nur partiell durch die gesonderte stabilitätssteigernde Behandlung des ausgewählten Teils der Oberfläche mechanisch stabilisiert ist, kann in bestimmten Grenzen, je nach den, z.B. klinischen, Anforderungen variieren. Bevorzugt betrifft die vorliegende Erfindung daher bioprosthetische bzw. biologische Materialien, und speziell eine biologische Herzklappe, insbesondere ein biologisches Herzklappensegel, basierend auf biologischem Material, das sich dadurch auszeichnet, dass darin die Zonen mit gesteigerter mechanischer Stabilität, jeweils bezogen auf die gesamte Oberfläche der biologischen Herzklappe bzw. insbesondere des Herzklappensegels als 100%, einen Anteil von bis zu ≤ 30%, insbesondere von ≤ 20%, vorzugsweise von 5 bis 20%, besonders bevorzugt von 10 bis 20%, der Oberfläche ausmachen. In einer bevorzugten Variante dieser Ausgestaltung der Erfindung liegt ein bioprosthetisches bzw. biologisches Material vor, und speziell eine biologische Herzklappe, insbesondere ein biologisches Herzklappensegel, basierend auf biologischem Material, das sich dadurch auszeichnet, dass darin die Eigenschaften der übrigen Zonen, jeweils bezogen auf die gesamte Oberfläche der biologischen Herzklappe bzw. insbesondere des Herzklappensegels als 100%, mit einem Anteil von ≥ 70%, insbesondere von ≥ 80%, vorzugsweise von 95 bis 80%, besonders bevorzugt von 90 bis 80%, der Oberfläche vor und nach der stabilitätssteigernden Behandlung im Wesentlichen unverändert sind.

Gemäß einer Ausgestaltung der Erfindung zeichnet sich diese dadurch aus, dass es sich bei dem biologischen Material, speziell der biologischen Herzklappe, insbesondere bei dem biologischen Herzklappensegel, basierend auf biologischem Material, um natives biologisches Material handelt.

Als "natives" Material wird hierbei ein biologisches Gewebe verstanden, das ohne eine weitreichende Vorbehandlung an dessen Oberfläche im Wesentlichen nur einer erfindungsgemäßen partiellen stabilitätssteigernden Behandlung der Oberfläche zur Erzielung von einer oder mehreren Zonen mit gesteigerter mechanischer Stabilität unterworfen wird. Beispielsweise kann es sich um eine biologische Herzklappe, insbesondere ein biologisches Herzklappensegel, basierend auf biologischem Material, handeln, das sich dadurch auszeichnet, dass das native biologische Material ein Kollagenbasiertes (-enthaltendes) biologisches Material ist.

Eine andere bevorzugte Ausgestaltung der Erfindung zeichnet sich dadurch aus, dass es sich bei dem biologischen Material, speziell der biologischen Herzklappe und insbesondere dem biologischen Herzklappensegel, basierend auf biologischem Material, um biologische Material handelt, das ein, vor einer erfindungsgemäßen partiellen stabilitätssteigernden Behandlung der Oberfläche zur Erzielung von einer oder mehreren Zonen mit gesteigerter mechanischer Stabilität, durch eine chemische Vorbehandlung bereits modifiziertes biologisches Material, vorzugsweise ein durch Dezellularisierung modifiziertes biologisches Material, ist. Beispielsweise kann es sich in dieser Ausgestaltung der Erfindung um eine biologische Herzklappe, insbesondere ein biologisches Herzklappensegel, basierend auf biologischem Material handeln, das sich dadurch auszeichnet, dass das durch chemische Vorbehandlung bereits modifizierte, vorzugsweise durch Dezellularisierung modifizierte biologische Material, ein durch Behandlung (chemische Vernetzung) mit einer, üblicherweise wässrigen, Glutaraldehydlösung, vorzugsweise durch Behandlung mit einer 0,3 bis 1,0 gew.-%igen Glutaraldehydlösung auf Basis einer auf einen pH von 7,2 bis 7,5 Phosphat-gepufferten isotonischen Kochsalzlösung, modifiziertes und/oder dezellularisiertes biologisches Material ist. Speziell kann die Glutaraldehydlösung bzw. deren Konzentrationsbereich somit z.B. als eine 0,3 bis 1 gew.-%ige Phosphat-gepufferte isotonische Kochsalzlösung (PBS 50 mM Phosphat) charakterisiert werden; ein bevorzugter pH-Wert ist dabei der pH von 7,38. Wie bereits weiter oben allgemein ausgeführt, wird das bioprosthetische bzw. biologische Material gemäß der vorliegenden Erfindung nur partiell, d.h. in bestimmten ausgewählten Bereichen oder Zonen durch eine gesonderte stabilitätssteigernde chemische Behandlung, mechanisch stabilisiert. Die gesonderte stabilitätssteigernde Behandlung des ausgewählten Teils der Oberfläche von bioprosthetischen bzw. biologischen Materialien, insbesondere von bioprosthetischen bzw. biologischen Herzklappen und speziell von bioprosthetischen bzw. biologischen Herzklappensegeln, kann gemäß der vorliegenden Erfindung in vielfältiger Weise erfolgen. Beispielsweise kann die gesonderte, partielle Behandlung der Oberfläche nach jedem dem Fachmann geläufigen Verfahren zur Behandlung von bioprosthetischen Materialien, speziell Verfahren zur Behandlung von biologischen Herzklappen und insbesondere von biologischen Herzklappensegeln, erfolgen. Die Behandlung wird hierbei so ausgeführt, dass nur diejenigen Bereiche selektiv einer stabilitätssteigernden Behandlung unterzogen werden, für die jeweils eine gesteigerte mechanische Stabilität erwünscht ist. Wird natives Material eingesetzt, werden demgemäß bevorzugt nur solche ausgewählten Bereiche, z.B. Randbereiche und/oder Nähbereiche, selektiv in diesen Bereichen durch chemische Vernetzung und/oder thermische Einwirkung, einer selektiv stabilitätssteigernden Behandlung unterworfen. Wird ein bereits vorbehandeltes biologisches Material, speziell eine bereits vorbehandelte biologische Herzklappe und insbesondere ein bereits vorbehandeltes biologisches Herzklappensegel, eingesetzt, können demgemäß bevorzugt solche ausgewählten Bereiche, z.B. Randbereiche oder Nähbereiche, selektiv in diesen Bereichen durch chemische Vernetzung einer zusätzlichen stabilitätssteigernden Behandlung unterworfen werden. Gemäß der Erfindung gelingt es also, die Oberfläche von nativen oder bereits vorbehandelten biologischen Materialien, speziell von nativen oder bereits vorbehandelten biologischen Herzklappen und insbesondere von nativen oder bereits vorbehandelten biologischen Herzklappensegeln, in gewünschter Weise örtlich differenziert zu modifizieren und an die örtlichen Anforderungen in den erfindungsgemäß behandelten Bereichen bzw. Zonen anzupassen.

Die erfindungsgemäße gesonderte stabilitätssteigernde Behandlung in ausgewählten Bereichen der Oberfläche von nativen oder bereits vorbehandelten biologischen Materialien, speziell von nativen oder bereits vorbehandelten biologischen Herzklappen und insbesondere von nativen oder bereits vorbehandelten biologischen Herzklappensegeln, kann beispielsweise, jedoch nicht einschränkend, nach den unten in den Beispielen angegebenen Methoden erfolgen. Diese Methoden dienen hiermit auch zur Herstellung der nachfolgenden erfindungsgemäßen bioprosthetischen Materialien, speziell von nativen oder von bereits vorbehandelten biologischen Herzklappen und insbesondere von nativen oder von bereits vorbehandelten biologischen Herzklappensegeln, bei denen die Oberfläche aufgrund einer gesonderten stabilitätssteigernden Behandlung nur eines Teils, insbesondere eines bestimmten ausgewählten Teils, der Oberfläche eine oder mehrere Zonen mit einer, im Vergleich zur mechanischen Stabilität vor der stabilitätssteigernden Behandlung, gesteigerten mechanischen Stabilität wie oben definiert aufweist und wobei die Eigenschaften der übrigen Zonen vor und nach der gesonderten stabilitätssteigernden Behandlung im Wesentlichen unverändert sind.

Wird natives Material eingesetzt, werden demgemäß nur solche ausgewählten Bereiche, z.B. Randbereiche oder Nähbereiche, selektiv in diesen Bereichen einer der nachfolgend beschriebenen gesonderten stabilitätssteigernden Behandlung unterworfen. Wird ein bereits vorbehandeltes biologisches Material, speziell eine bereits vorbehandelte biologische Herzklappe und insbesondere ein bereits vorbehandeltes biologisches Herzklappensegel, eingesetzt, können demgemäß solche ausgewählten Bereiche, z.B. Randbereiche oder Nähbereiche, selektiv in diesen Bereichen einer, gesonderten zusätzlichen stabilitätssteigernden Behandlung unterworfen werden. Diese gesonderte bzw. gesonderte zusätzliche Behandlung ist eine selektive Behandlung der ausgewählten Bereiche, z.B. Randbereiche oder Nähbereiche mit. Divinylsulfon und einer Mischung aus einem verzweigten Polyethylenamin und Divinylsulfon;

In der bevorzugten Ausgestaltung im Zusammenhang mit der gesonderten chemischen stabilitätssteigernden Behandlung ausgewählter Bereiche, z.B. Randbereiche oder Nähbereiche, von bioprosthetischen Materialien, betrifft die Erfindung eine biologische Herzklappe, insbesondere ein Herzklappensegel, basierend auf biologischem Material, wie oben beschrieben, worin die Oberfläche der biologischen Herzklappe, insbesondere des Herzklappensegels, eine oder mehrere Zonen mit gesteigerter mechanischer Stabilität aufweist, bei der die gesteigerte mechanische Stabilität auf einer gesonderten stabilitätssteigernden Behandlung der Oberfläche dieser Zone mit Divinylsulfon und einer Mischung aus einem verzweigten Polyethylenamin und Divinylsulfon beruht.

Bei der Herstellung von biologischem Herzklappenmaterial hat sich gezeigt, dass das Ausschneiden unter Verwendung eines Lasers bessere Resultate bringt, als wenn das Material mechanisch geschnitten oder gestanzt wird. Bessere Resultate bedeutet hierbei, dass ebenfalls die Randbereiche stabiler gestaltet sind. Grund dafür ist die Wärmeeinwirkung des Lasers auf das Material. Biologisches Herzklappenmaterial kann somit vorteilhaft durch Ausschneiden unter Verwendung eines Lasers geschnitten werden. Durch den Laser werden aufgrund der Wärmeeinwirkung des Lasers vorteilhaft stabilisierte Randbereiche gestaltet. Das Laserschneiden kann mit einer darunterliegenden Matrix erfolgen, wobei die Matrix gewünschtenfalls mitgeschnitten wird oder nur als inerte Auflage für das mit dem Laser auszuschneidende biologische Material dient. Als Matrix dienen thermisch schlecht leitende Materialien. Als thermisch schlecht leitende Matrixmaterialien kommen Kunststoffe, bevorzugt das Polyimid, in Frage. Polyimide (Kurzzeichen PI) sind Hochleistungskunststoffe, deren wichtigstes Strukturmerkmal die Imidgruppe ist. Als weitere mögliche thermisch schlecht leitende Kunststoffe kommen u.a. auch Polybismaleinimid (PBMI), Polybenzimidazol (PBI) und Polyoxadiazobenzimidazol (PBO), Polyimidsulfon (PISO) und Polymethacrylimid (PMI) in Frage. Alternativ kann die unter dem biologischen Material befindliche Matrix auch nicht mit getrennt werden. Auch dann wird ein schlecht leitfähiges, jedoch inertes Matrixmaterial wie beispielsweise Glas benutzt. Durch die geschickte Auswahl der Matrix kann die durch den Laser eingebrachte Wärmemenge besser auf das Gewebe übertragen werden. Dabei wird der Laserfokus so eingestellt, dass es zu einer Denaturierung des Randes kommt. Eine vollständige Denaturierung soll allerdings vermieden werden.

Die Erfindung stellt bioprosthetische Materialien mit vorteilhaften Eigenschaften zur Verfügung, die hier am Beispiel von Herzklappen, insbesondere am Beispiel von Herzklappensegeln, weiter erläutert werden sollen. Bei einer Herzklappe erfahren die Klappensegel unterschiedliche mechanische Herausforderungen. Ebenso wie künstliche Herzklappen werden auch biologische Herzklappen zum Einnähen mit einer Polyestermanschette umgeben. Gemäß der vorliegenden Erfindung kann nun durch die flächenmäßig begrenzte und/oder punktuelle Optimierung der Bereiche das bioprosthetische oder biologische Material für den jeweiligen speziellen Einsatzzweck maßgeschneidert vorbereitet werden. Durch die erfindungsgemäße örtlich begrenzte Behandlung wird das bioprosthetische oder biologische Material auf die örtlich unterschiedlichen Belastungen angepasst. Das so gestaltete bioprosthetische oder biologische Material lässt sich z.B. besser vernähen. Durch den erfindungsgemäß selektiv verstärkten Nahtbereich reißen Fäden nicht mehr aus. Die ganze Herzklappe lässt sich leichter auf einen geringeren Radius crimpen. Das Perikard der erfindungsgemäß behandelten Herzklappe ist auf der Fläche weicher, nur die Ränder sind vernetzt und damit fester. Druckstellen treten nicht mehr auf. Das bioprosthetische oder biologische Material ist elastischer, Abdrücke durch das Crimpen bilden sich leichter zurück, Vorverletzungen, an denen das bioprosthetische oder biologische Material versagen könnte, treten nicht mehr auf. Für den klinischen Einsatz wird das eigentliche Herzklappengewebe auf einem Gerüst ("Stent") befestigt oder gerüstfrei verwendet.

Durch die Erfindung wird somit ein Beitrag zur vermehrten Einsetzbarkeit von biologischen Herzklappen, insbesondere biologischer Herzklappensegel, geleistet. Durch die vorliegende Erfindung wird Stabilität des Herzklappensegels verbessert, ohne dass das gesamte bioprosthetische oder biologische Material chemisch oder thermisch verändert wird. Vorzugsweise werden nur die Bereiche zusätzlich stabilisiert, bei denen es erfahrungsgemäß zu Rissen oder Materialermüdungen kommt. Durch die Verstärkung der erfindungsgemäßen biologischen Herzklappe an den zweckmäßigen Stellen, bleibt der Hauptanteil der Oberfläche chemisch oder thermisch unbeeinflusst und somit körperverträglicher. Ein weiterer Vorteil der vorliegenden Erfindung besteht darin, dass das biologische Material den Anforderungen entsprechend angepasst werden kann. So kann es an den Rändern, an denen das Material mit dem Frame (Rahmen) oder Stent (Gerüst) vernäht werden soll, besonders verstärkt werden, um ein besonders großes Nahtrückhaltevermögen zu erreichen.

Demgemäß betrifft die Erfindung auch bioprosthetische Materialien mit verbesserten Materialeigenschaften, wie insbesondere verbesserte Rissfestigkeit und/oder die schon definierten Materialkennwerte, wie oben angegeben, aufweisen.

Die Erfindung betrifft daher auch eine biologische Herzklappe, insbesondere ein Herzklappensegel, basierend auf biologischem Material, wie oben beschrieben, worin die biologische Herzklappe, insbesondere das Herzklappensegel, eine der folgenden Materialeigenschaften (z.B. Rissfestigkeit) aufweist:
a) SRS von 30 bis 450 N/mm², vorzugsweise von 50 bis 300 N/mm²;
b) SRS weiter bevorzugt 100 bis 200 N/mm², besonders bevorzugt 150 bis 200 N/mm².

Die bevorzugten erfindungsgemäßen bioprosthetischen Materialien, speziell die biologischen Herzklappen und insbesondere die biologischen Herzklappensegel, lassen sich, wie oben bereits im Zusammenhang mit deren Beschreibung dargestellt, durch eine gesonderte Behandlung bestimmter ausgewählter Teile der Oberfläche gewinnen. Die Erfindung betrifft somit auch ein Verfahren zur Herstellung einer biologischen Herzklappe, insbesondere eines biologischen Herzklappensegels, wie oben beschrieben und definiert, wobei die biologische Herzklappe, insbesondere das biologische Herzklappensegel, auf einem nativen oder ggf. bereits modifizierten biologischen Material basiert und dessen Oberfläche eine oder mehrere Zonen mit einer gesteigerten mechanischen Stabilität aufweist, und sich dadurch auszeichnet, dass man nur einen (ausgewählten) Teil der Oberfläche der biologischen Herzklappe, insbesondere des biologischen Herzklappensegels, einer gesonderten stabilitätssteigernden chemischen Behandlungder Oberfläche dieser Zone mit Divinylsulfon und einer Mischung aus einem verzweigten Polyethylenamin und Divinylsulfon unterwirft, um eine oder mehrere Zonen mit einer, im Vergleich zur mechanischen Stabilität vor der stabilitätssteigernden Behandlung, gesteigerten mechanischen Stabilität zu erzeugen, und wobei, jeweils bezogen auf die gesamte Oberfläche der biologischen Herzklappe bzw. insbesondere des Herzklappensegels als 100%, die Zonen mit gesteigerter mechanischer Stabilität mit maximal einem Anteil von bis zu 40% der Oberfläche gebildet werden und die Eigenschaften der übrigen Zonen mit einem Anteil von wenigstens 60% der Oberfläche durch die stabilitätssteigernde Behandlung im Wesentlichen nicht verändert werden.

### Beispiele

Für moderne biologische Herzklappensegel ist eine Dezellularisierung unbedingt erforderlich. Verfahren zur Dezellularisierung sind dem Fachmann hinreichend bekannt. Die chemische Vernetzung durch Glutaraldehyd ist ebenfalls ein eingeführtes, bewährtes Verfahren. Abweichend dazu beschreibt die vorliegende Erfindung eine, gegebenenfalls nur zusätzliche, (flächenbezogene) partielle chemische Vernetzung der biologischen Herzklappensegel mit Glutaraldehyd. Des Weiteren werden als Beispiele weitere chemische Methoden zur, gegebenenfalls nur zusätzlichen, (flächenbezogenen) partiellen chemischen oder auch thermischen Vernetzung der biologischen Herzklappensegel angegeben. Die biologischen Materialien der vorliegenden Erfindung zeichnen sich durch mechanische Unterschiede zwischen Randbereich und übriger Fläche im biologischen Material aus. Hierbei kann auch ein wie im Stand der Technik bereits komplett vernetztes und fixiertes biologisches Material, z.B. ein Gewebe, das analog wie in Beispiel 1 zunächst komplett vernetzt und fixiert wurde, eingesetzt werden, wobei gemäß der vorliegenden Erfindung aber z.B. der Randbereich oder auch jeder andere gewünschte Bereich des biologischen Materials noch eine erfindungsgemäße gesonderte zusätzliche Behandlung erfährt.

Soweit nicht anders angegeben, handelt es sich bei den in den folgenden Beispielen angegebenen %-Angaben um Gew.-%-Angaben.

### Ausführungsbeispiel 1:

### Stabilisierung des Randbereichs durch Behandlung mit Divinylsulfon und einer Mischung aus einem verzweigten Polyethylenimin und Divinylsulfon

Die Zugabe von 0,1%ige KOH bescheunigt die Vernetzungsreaktion für das Material aus Beispiel 2. Divinylsulfon wird als 1 bis 11%ige wässrige Lösung eingesetzt. Bevorzugt ist eine 5-10%ige Lösung. Die Randbereiche werden in diese Lösung für 15 Min getaucht und dann entnommen. Alternativ kann der Randbereich auch mit einer 10%igen DVS/Wasserlösung besprüht werden. Werden 0,1%ige KOH zugegeben, muss die Lösung auf Eis gekühlt werden. Nach dem Aufsprühen wird das Gewebe auf 40°C für 30 Min erhitzt.

Nach dieser Behandlung ist das Material schon am Rand stabilisiert. Eine Weiterbehandlung mit Polyethylenimin führt noch zu einer weiteren Verfestigung, wie sie insbesondere für den Nahtbereich sinnvoll ist. Dazu wird nach dem Tauchen oder Aufsprühen das Gewebe in Polyethylenimin getaucht. Und nach dem Entnehmen und Abtupfen für 30 Min bei 40 °C inkubiert.

### (Vergleichsbeispiel, nicht unter den Umfang der Erfindung fallend)

Das vorvernetzte Gewebe aus Beispiel 2 wird am Randbereich mit einer Naht versehen. Dabei wird der Rand mit einem geeigneten Faden vernäht. Verschiedene Nähtechniken sind aus der Textilindustrie bekannt und können angewendet werden. Als Nahtmaterial kann ein Monofilament oder ein Multifilament eingesetzt werden. Beispielsweise TEVDEK II^{®} von Deknatel oder PROFILEN^{®} von Lenzing. Pures Teflon hat sich aufgrund seines geringen Reibwertes beim Nähen besonders bewährt.

## Patentansprüche

1. Eine bioprosthetische Komponente für ein Implantat, basierend auf einem nativen oder ggf. bereits modifizierten biologischen Material, **dadurch gekennzeichnet, dass** die Oberfläche des biologischen Materials aufgrund einer gesonderten stabilitätssteigernden Behandlung nur eines Teils der Oberfläche eine oder mehrere Zonen mit einer, im Vergleich zur mechanischen Stabilität vor der stabilitätssteigernden Behandlung, gesteigerten mechanischen Stabilität aufweist, und wobei, jeweils bezogen auf die gesamte Oberfläche der bioprosthetischen Komponente als 100%, die Zonen mit gesteigerter mechanischer Stabilität einen Anteil ≤ 40% der Oberfläche ausmachen und die Eigenschaften der übrigen Zonen mit einem Anteil von ≥ 60% der Oberfläche vor und nach der stabilitätssteigernden Behandlung im Wesentlichen unverändert sind, wobei die gesteigerte mechanische Stabilität auf einer gesonderten stabilitätssteigernden Behandlung der Oberfläche dieser Zonen beruht, die eine stabilitätssteigernde chemische Behandlung ist, wobei die gesteigerte mechanische Stabilität auf einer gesonderten stabilitätssteigernden Behandlung der Oberfläche dieser Zone mit Divinylsulfon und einer Mischung aus einem verzweigten Polyethylenamin und Divinylsulfon beruht.

2. Eine bioprosthetische Komponente für ein Implantat nach Anspruch 1, **dadurch gekennzeichnet, dass** das Implantat ausgewählt ist aus der Gruppe bestehend aus (1) Closure Devices, vorzugsweise Aneurysmen-Stents für den gesamten Blutkreislauf einschließlich des Blutkreislaufs im Hirnbereich; (2) Klappensystemen, vorzugsweise biologische Herzklappen und Venenklappen, besonders bevorzugt biologische Herzklappen ausgewählt aus der Gruppe bestehend aus Herzklappensegeln, Mitral-Herzklappen, Aorten-Herzklappen oder Pulmonar-Herzklappen; und (3) Gewebesegeln zum Abdichten von Organnarben, vorzugsweise Herzsegel zum Abdichten von Narben am Herzen, besonders bevorzugt Herzsegel zum Abdichten von Narben am Herzen bei einem atrio-ventrikulären Septumdefekt, einem Atriumseptumsefekt oder Ventrikelseptumdefekt.

3. Eine bioprosthetische Komponente für ein Implantat nach Anspruch 1 oder 2, **dadurch gekennzeichnet, dass** die Oberfläche der bioprosthetischen Komponente eine oder mehrere Zonen mit gesteigerter mechanischer Stabilität aufweist, wobei eine solche Zone bei zweckentsprechender Verwendung der bioprosthetischen Komponente einer erhöhten mechanischen Dauerbelastung unterliegt, und wobei vorzugsweise diese Zone wenigstens eine Kante und/oder Nahtrand und/oder Nahtbereich und/oder sonstiger Fixierungsbereich der bioprosthetischen Komponente oder eine Kombination dieser Zonen ist, und wobei besonders bevorzugt diese Zone wenigstens ein Nahtrand oder Nahtbereich der bioprosthetischen Komponente ist.

4. Eine bioprosthetische Komponente für ein Implantat nach einem der Ansprüche 1 bis3, **dadurch gekennzeichnet, dass** darin die Zonen mit gesteigerter mechanischer Stabilität, jeweils bezogen auf die gesamte Oberfläche der bioprosthetischen Komponente als 100%, einen Anteil von ≤ 30%, insbesondere von ≤ 20%, vorzugsweise einen Anteil von 5 bis 20%, besonders bevorzugt einen Anteil von 10 bis 20%, der Oberfläche ausmachen.

5. Eine bioprosthetische Komponente für ein Implantat nach einem der Ansprüche 1 bis 3, **dadurch gekennzeichnet, dass** darin die Eigenschaften der übrigen Zonen, jeweils bezogen auf die gesamte Oberfläche der der bioprosthetischen Komponente als 100%, mit einem Anteil von ≥ 70%, insbesondere ≥ 80%, vorzugsweise von 95 bis 80%, besonders von 90 bis 80%, der Oberfläche des Herzklappensegels vor und nach der stabilitätssteigernden Behandlung im Wesentlichen unverändert sind.

6. Eine bioprosthetische Komponente für ein Implantat nach einem der Ansprüche 1 bis 5, **dadurch gekennzeichnet, dass** das biologische Material ein durch chemische Vorbehandlung bereits modifiziertes biologisches Material, vorzugsweise ein durch Dezellularisierung modifiziertes biologisches Material, ist.

7. Eine bioprosthetische Komponente für ein Implantat nach einem der Ansprüche 1 bis6, **dadurch gekennzeichnet, dass** das Implantat eine biologische Herzklappe, insbesondere ein Herzklappensegel, basierend auf einem nativen oder ggf. bereits modifizierten biologischen Material, ist; vorzugsweise eine biologische Herzklappe, insbesondere ein Herzklappensegel, basierend auf einem nativen oder ggf. bereits modifizierten biologischen Material, worin das biologische Material ein durch chemische Vorbehandlung bereits modifiziertes biologisches Material, besonders bevorzugt ein durch Dezellularisierung modifiziertes biologisches Material, ist.

8. Eine bioprosthetische Komponente für ein Implantat nach Anspruch 7, **dadurch gekennzeichnet, dass** die bioprosthetische Komponente, vorzugsweise eine biologische Herzklappe, insbesondere ein Herzklappensegel, auf einem biologischen Material basiert, wobei das durch chemische Vorbehandlung bereits modifizierte, vorzugsweise durch Dezellularisierung modifizierte biologische Material, ein durch Behandlung mit einer Glutaraldehydlösung, vorzugsweise durch Behandlung mit einer 0,3 bis 1,0 gew.-%igen Glutaraldehydlösung auf Basis einer auf einen pH von 7,2 bis 7,5 Phosphat-gepufferten isotonischen Kochsalzlösung, modifiziertes und/oder dezellularisiertes biologisches Material ist.

9. Eine bioprosthetische Komponente für ein Implantat nach wenigstens einem der Ansprüche 1 bis8zu, **dadurch gekennzeichnet, dass** die bioprosthetische Komponente, vorzugsweise eine biologische Herzklappe, insbesondere ein Herzklappensegel, auf einem biologischem Material basiert, und wobei die Oberfläche der bioprosthetischen Komponente, vorzugsweise der biologischen Herzklappe, insbesondere des Herzklappensegels, eine oder mehrere Zonen mit gesteigerter mechanischer Stabilität aufweist..

10. Eine bioprosthetische Komponente für ein Implantat nach wenigstens einem der Ansprüche 1 bis 9, **dadurch gekennzeichnet, dass** die bioprosthetische Komponente, vorzugsweise eine biologische Herzklappe, insbesondere ein Herzklappensegel, auf einem biologischem Material basiert, und wobei die Oberfläche der bioprosthetischen Komponente, vorzugsweise der biologischen Herzklappe, insbesondere des Herzklappensegels, eine oder mehrere Zonen mit gesteigerter mechanischer Stabilität aufweist, wobei eine solche Zone folgende Materialeigenschaft und/oder Rissfestigkeit aufweist: SRS von 30 bis 450 N/mm², vorzugsweise von 50 bis 300 N/mm², weiter bevorzugt von 100 bis 200 N/mm², besonders bevorzugt von 150 bis 200 N/mm².

11. Verfahren zur Herstellung einer bioprosthetischen Komponente für ein Implantat, vorzugsweise einer biologischen Herzklappe, insbesondere eines Herzklappensegels, wie in den Ansprüchen 1 bis 10 definiert, wobei die bioprosthetische Komponente für ein Implantat, vorzugsweise die biologische Herzklappe, insbesondere das Herzklappensegel, auf einem nativen oder ggf. bereits modifizierten biologischen Material basiert und wobei die Oberfläche der bioprosthetischen Komponente für ein Implantat, vorzugsweise der biologische Herzklappe, insbesondere des Herzklappensegels, eine oder mehrere Zonen mit einer gesteigerten mechanischen Stabilität aufweist, **dadurch gekennzeichnet, dass** man nur einen Teil der Oberfläche der bioprosthetischen Komponente für ein Implantat, vorzugsweise der biologischen Herzklappe, insbesondere des Herzklappensegels, einer gesonderten stabilitätssteigernden chemischen Behandlung der Oberfläche dieser Zone mit Divinylsulfon und einer Mischung aus einem verzweigten Polyethylenamin und Divinylsulfon unterwirft, um eine oder mehrere Zonen mit einer, im Vergleich zur mechanischen Stabilität vor der stabilitätssteigernden Behandlung, gesteigerten mechanischen Stabilität zu erzeugen,
und wobei, jeweils bezogen auf die gesamte Oberfläche der bioprosthetischen Komponente für ein Implantat bzw. vorzugsweise der biologischen Herzklappe bzw. insbesondere des Herzklappensegels als 100%,
die Zonen mit gesteigerter mechanischer Stabilität mit maximal einem Anteil von bis zu 40% der Oberfläche gebildet werden und die Eigenschaften der übrigen Zonen mit einem Anteil von wenigstens 60% der Oberfläche durch die stabilitätssteigernde Behandlung im Wesentlichen nicht verändert werden.

## Claims

1. A bioprosthetic component for an implant, based on a native or possibly already modified biological material, **characterised in that**, due to a separate stability-increasing treatment of just part of the surface, the surface of the biological material has one or more zones having an increased mechanical stability compared to the mechanical stability before the stability-increasing treatment, and wherein the zones having increased mechanical stability account for a proportion of ≤ 40 % of the surface and the properties of the remaining zones accounting for a proportion of ≥ 60 % of the surface are substantially unaltered before and after the stability-increasing treatment, in each case based on the total surface of the bioprosthetic component as 100 %, wherein the increased mechanical stability is based on a separate stability-increasing treatment of the surface of these zones which is a stability increasing chemical treatment, wherein the increased mechanical stability is based on a separate stability-increasing treatment of the surface of this zone with divinyl sulfone and a mixture of a branched polyethylene amine and divinyl sulfone.

2. The bioprosthetic component for an implant according to Claim 1, **characterised in that** the implant is selected from the group consisting of (1) closure devices, preferably aneurysm stents for the entire blood circulation system, including the blood circulation system in a region of the brain; (2) valve systems, preferably biological heart valves and venous valves, particularly preferably biological heart valves selected from the group consisting of heart valve leaflets, mitral heart valves, aortic heart valves or pulmonary heart valves; and (3) tissue leaflets for sealing organ scarring, preferably heart leaflets for sealing scarring in the heart, particularly preferably heart valves for sealing scarring in the heart in case of an atrioventricular septal defect, an atrial septal defect, or a ventricular septal defect.

3. The bioprosthetic component for an implant according to Claim 1 or 2, **characterised in that** the surface of the bioprosthetic component has one or more zones with increased mechanical stability, wherein such zone is subjected to increased continuous mechanical stressing with appropriate use of the bioprosthetic component, and wherein this zone is preferably at least an edge and/or stitching edge and/or stitching region and/or other fixing region of the bioprosthetic component or a combination thereof, and wherein this zone is particularly preferably at least one stitching edge or stitching region of the bioprosthetic component.

4. The bioprosthetic component for an implant according to one of Claims 1 to 3, **characterised in that** the zones with increased mechanical stability, in each case based on the total surface of the bioprosthetic component as 100 %, account for a proportion of ≤ 30 %, in particular of ≤ 20 %, preferably a proportion of from 5 to 20 %, particularly preferably a proportion of from 10 to 20 %, of the surface.

5. The bioprosthetic component for an implant according to one of Claims 1 to 3, **characterised in that** the properties of the remaining zones having a proportion of ≥ 70 %, in particular ≥ 80 %, preferably from 95 to 80 %, particularly from 90 to 80 %, of the surface of the heart valve leaflet, in each case based on the total surface of the bioprosthetic component as 100 %, are substantially unaltered before and after the stability-increasing treatment.

6. The bioprosthetic component for an implant according to one of Claims 1 to 5, **characterised in that** the biological material is a biological material already modified by chemical pre-treatment, preferably a biological material modified by decellularisation.

7. The bioprosthetic component for an implant according to one of Claims 1 to 6, **characterised in that** the implant is a biological heart valve, in particular a heart valve leaflet, based on a native or possibly already modified biological material; preferably a biological heart valve, in particular a heart valve leaflet, based on a native or possibly already modified biological material, wherein the biological material is a biological material already modified by chemical pre-treatment, particularly preferably a biological material modified by decellularisation.

8. The bioprosthetic component for an implant according to Claim 7, **characterised in that** the bioprosthetic component, preferably a biological heart valve, in particular a heart valve leaflet, is based on a biological material, wherein the biological material already modified by chemical pre-treatment, preferably modified by decellularisation, is a biological material modified and/or decellularised by treatment with a glutaraldehyde solution, preferably by treatment with a 0.3 to 1.0 % by weight glutaraldehyde solution based on a phosphate-buffered isotonic sodium chloride solution having a pH of from 7.2 to 7.5.

9. The bioprosthetic component for an implant according to at least one of Claims 1 to 8, **characterised in that** the bioprosthetic component, preferably a biological heart valve, in particular a heart valve leaflet, is based on a biological material, and wherein the surface of the bioprosthetic component, preferably of the biological heart valve, in particular of the heart valve leaflet, has the one or more zones having increased mechanical stability.

10. The bioprosthetic component for an implant according to at least one of Claims 1 to 9, **characterised in that** the bioprosthetic component, preferably a biological heart valve, in particular a heart valve leaflet, is based on a biological material, and wherein the surface of the bioprosthetic component, preferably of the biological heart valve, in particular of the heart valve leaflet, has one or more zones having increased mechanical stability, wherein such a zone has the following material property and/or tear resistance: SRS of from 30 to 450 N/mm², preferably from 50 to 300 N/mm², more preferably from 100 to 200 N/mm², particularly preferably from 150 to 200 N/mm².

11. A method for producing a bioprosthetic component for an implant, preferably a biological heart valve, in particular a heart valve leaflet, as defined in Claims 1 to 10, wherein the bioprosthetic component for an implant, preferably the biological heart valve, in particular the heart valve leaflet, is based on a native or possibly already modified biological material, and wherein the surface of the bioprosthetic component for an implant, preferably of the biological heart valve, in particular of the heart valve leaflet, has one or more zones having an increased mechanical stability, **characterised in that** only part of the surface of the bioprosthetic component for an implant, preferably of the biological heart valve, in particular of the heart valve leaflet, is subjected to a separate stability-increasing chemical treatment of the surface of this zone with divinyl sulfone and a mixture of a branched polyethylene amine and divinyl sulfone, so as to produce one or more zones having an increased mechanical stability compared to the mechanical stability before the stability-increasing treatment,
and wherein, in each case based on the total surface of the bioprosthetic component for an implant, or preferably of the biological heart valve, or in particular of the heart valve leaflet, as 100 %,
the zones with increased mechanical stability are formed by a maximum proportion of up to 40 % of the surface and the properties of the remaining zones with a proportion of at least 60 % of the surface remain substantially unaltered by the stability-increasing treatment.

## Revendications

1. Composant bioprothétique pour un implant, à base d'un matériau biologique natif ou éventuellement déjà modifié, **caractérisé en ce que** la surface du matériau biologique présente, suite à un traitement distinct pour le renforcement de la stabilité d'une partie seulement de la surface, une ou plusieurs zones avec une stabilité mécanique renforcée en comparaison avec la stabilité mécanique avant le traitement pour le renforcement de la stabilité, et dans lequel, respectivement par rapport à la surface totale du composant bioprothétique en tant que 100%, les zones dont la stabilité mécanique a été renforcée représentent une part ≤ 40% de la surface, et les propriétés des autres zones avec une part de ≥ 60% de la surface demeurent quasiment inchangées avant et après le traitement pour le renforcement de la stabilité, dans lequel la stabilité mécanique renforcée repose sur un traitement distinct pour le renforcement de la stabilité de la surface de ces zones, lequel est un traitement chimique pour le renforcement de la stabilité, dans lequel la stabilité mécanique renforcée repose sur un traitement distinct pour le renforcement de la stabilité de la surface de cette zone avec du sulfone divinylique et un mélange entre une polyéthylèneamine ramifiée et du sulfone divinylique.

2. Composant bioprothétique pour un implant selon la revendication 1, **caractérisé en ce que** l'implant est sélectionné parmi le groupe comprenant (1) des dispositifs de fermeture, de préférence des endoprothèses d'anévrisme pour l'ensemble du circuit sanguin, y compris le circuit sanguin dans la zone cérébrale ; (2) des systèmes de valve, de préférence des valves cardiaques ou veineuses biologiques, ou mieux, des valves cardiaques biologiques sélectionnées parmi le groupe comprenant des feuillets de valve cardiaque, des valves cardiaques mitrales, des valves cardiaques aortiques ou des valves cardiaques pulmonaires ; et (3) des feuillets tissulaires pour l'isolation de cicatrices d'organes, de préférence des feuillets cardiaques pour l'isolation de cicatrices sur le coeur, ou mieux, des feuillets cardiaques pour l'isolation de cicatrices sur le coeur dans le cas d'un défaut auriculo-ventriculaire, d'un défaut inter-auriculaire ou d'un défaut inter-ventriculaire.

3. Composant bioprothétique pour un implant selon la revendication 1 ou 2, **caractérisé en ce que** la surface du composant bioprothétique présente une ou plusieurs zones avec une stabilité mécanique renforcée, dans lequel une telle zone est soumise à une charge mécanique permanente renforcée lors d'une utilisation conforme du composant bioprothétique, et dans lequel cette zone est un bord et/ou un bord de suture et/ou une région de suture et/ou une autre région de fixation du composant bioprothétique ou une combinaison de ces zones, et dans lequel, de façon particulièrement préférée, cette zone est au moins un bord de suture ou une région de suture du composant bioprothétique.

4. Composant bioprothétique pour un implant selon l'une des revendications 1 à 3, **caractérisé en ce que** dans celui-ci, les zones présentant une stabilité mécanique renforcée représentent une part ≤ 30%, en particulier ≤ 20%, de préférence une part de 5 à 20%, de façon particulièrement préférée une part de 10 à 20% de la surface, respectivement par rapport à la totalité de la surface du composant bioprothétique en tant que 100%.

5. Composant bioprothétique pour un implant selon l'une des revendications 1 à 3, **caractérisé en ce que** dans celui-ci, les propriétés des autres zones avec une part ≥ 70%, en particulier ≥ 80%, de préférence de 95 à 80%, de façon particulièrement préférée de 90 à 80% de la surface du feuillet de valve cardiaque, respectivement par rapport à la surface totale du composant bioprothétique en tant que 100%, demeurent quasiment inchangées avant et après le traitement pour le renforcement de la stabilité

6. Composant bioprothétique pour un implant selon l'une des revendications 1 à 5, **caractérisé en ce que** le matériau biologique est un matériau biologique déjà modifié par un prétraitement chimique, de préférence un matériau biologique modifié par décellularisation.

7. Composant bioprothétique pour un implant selon l'une des revendications 1 à 6, **caractérisé en ce que** l'implant est une valve cardiaque biologique, en particulier un feuillet de valve cardiaque, à base d'un matériau biologique natif ou éventuellement déjà modifié ; de préférence une valve cardiaque biologique, en particulier un feuillet de valve cardiaque, à base d'un matériau biologique natif ou éventuellement déjà modifié, où le matériau biologique est un matériau biologique déjà modifié par un prétraitement chimique, de façon particulièrement préférée un matériau biologique modifié par décellularisation.

8. Composant bioprothétique pour un implant selon la revendication 7, **caractérisé en ce que** le composant bioprothétique, de préférence une valve cardiaque biologique, en particulier un feuillet de valve cardiaque, est constitué à base d'un matériau biologique, dans lequel le matériau biologique déjà modifié par un prétraitement chimique, de préférence le matériau biologique modifié par décellularisation, est un matériau biologique modifié et/ou décellularisé par un traitement avec une solution de glutaraldéhyde, de préférence par un traitement avec une solution de glutaraldéhyde à raison de 0,3 à 1,0% en poids, à base d'une solution saline isotonique tamponnée au phosphate avec un pH de 7,2 à 7,5.

9. Composant bioprothétique pour un implant selon au moins l'une des revendications 1 à 8, **caractérisé en ce que** le composant bioprothétique, de préférence une valve cardiaque biologique, en particulier un feuillet de valve cardiaque, est constitué à base d'un matériau biologique, et dans lequel la surface du composant bioprothétique, de préférence de la valve cardiaque biologique, en particulier du feuillet de valve cardiaque, présente une ou plusieurs zones avec une stabilité mécanique renforcée.

10. Composant bioprothétique pour un implant selon au moins l'une des revendications 1 à 9, **caractérisé en ce que** le composant bioprothétique, de préférence une valve cardiaque biologique, en particulier un feuillet de valve cardiaque, est constitué à base d'un matériau biologique, et dans lequel la surface du composant bioprothétique, de préférence de la valve cardiaque biologique, en particulier du feuillet de valve cardiaque, présente une ou plusieurs zones avec une stabilité mécanique renforcée, dans lequel une telle zone présente les propriétés de matériau suivantes et/ou la résistance à la fissuration suivante : SRS de 30 à 450 N/mm², de préférence de 50 à 300 N/mm², ou mieux, de 100 à 200 N/mm², de façon particulièrement préférée de 150 à 200 N/mm².

11. Procédé pour la fabrication d'un composant bioprothétique pour un implant, de préférence une valve cardiaque biologique, en particulier un feuillet de valve cardiaque, tel que défini dans les revendications 1 à 10, dans lequel le composant bioprothétique pour un implant, de préférence la valve cardiaque biologique, en particulier le feuillet de valve cardiaque, est constitué à base d'un matériau biologique natif ou éventuellement déjà modifié, et dans lequel la surface du composant bioprothétique pour un implant, de préférence la valve cardiaque biologique, en particulier le feuillet de valve cardiaque, présente une ou plusieurs zones avec une stabilité mécanique renforcée, **caractérisé en ce que** seule une partie de la surface du composant bioprothétique pour un implant, de préférence de la valve cardiaque biologique, en particulier du feuillet de valve cardiaque, est soumise à un traitement chimique distinct pour le renforcement de la stabilité de la surface de cette zone avec du sulfone divinylique et un mélange entre une polyéthylèneamine ramifiée et du sulfone divinylique, afin de produire une ou plusieurs zones avec une stabilité mécanique renforcée en comparaison avec la stabilité mécanique avant le traitement pour le renforcement de la stabilité,
et dans lequel, respectivement par rapport à la surface totale du composant bioprothétique pour un implant ou de préférence de la valve cardiaque biologique, en particulier du feuillet de valve cardiaque, en tant que 100%, les zones avec une stabilité mécanique renforcée sont formées avec une part maximale jusqu'à 40% de la surface, et les propriétés des autres zones avec une part d'au moins 60% de la surface ne sont quasiment pas modifiées par le traitement pour le renforcement de la stabilité.
